# EUROPÄISCHE PATENTANMELDUNG

(11) **EP 0 831 259 A2**
(43) Veröffentlichungstag der Anmeldung: **25.03.1998**
(21) Anmeldenummer: 97111340.2
(22) Anmeldetag: 04.07.1997
(51) Int. Cl.: F16K 7/04, A61M 16/20

(54) **Flussregulator**

(30) Priorität: 12.09.1996 SE 9603313
(71) Anmelder: Siemens-Elema AB, 171 95 Solna (SE)
(72) Erfinder: Krahbichler, Erik, 170 71 Solna (SE); Eriksson, Per-Göran, 183 40 Täby (SE); Kock, Mikael, 184 91 Akersberga (SE); Bolmgren, Jan, 162 46 Vällingby (SE)

(57) **Zusammenfassung**

Die Erfindung betrifft einen Flußregulator, vorzugsweise bei einem Respirator/Ventilator, bei dem der Flußregulator einen Schlauch, der von einem Medium, dessen Fluß geregelt werden soll, durchströmt wird, und ein außerhalb des Schlauches angeordnetes Drosselventil umfaßt. Das Drosselventil umfaßt genau einander gegenüber angeordnete Druckmittel, zwischen denen der Schlauch anbringbar ist und die den Durchströmungsquerschnitt des Schlauches in einer ersten Endlage unbeeinflußt lassen und in einer zweiten Endlage den Schlauch ganz zusammenpressen. Um einen Flußregulator dieser Art mit einem Drosselventil zu erhalten, das den Schlauch schont und außerdem einen Schlauch mit einem verhältnismäßig großen Durchströmungsquerschnitt ganz zusammenpressen kann, wird erfindungsgemäß vorgeschlagen, daß die Druckmittel (6, 7, 15, 16, 35, 38) derart ausgebildet sind, daß sie bei einem Zusammenpressen des Schlauches (1), diesen von beiden Seiten aktiv zusammendrücken.

## Beschreibung

Die Erfindung betrifft einen Flußregulator, vorzugsweise bei einem Respirator/Ventilator, bei dem der Flußregulator einen Schlauch, der von einem Medium, dessen Fluß geregelt werden soll, durchströmt wird, und ein außerhalb des Schlauches angeordnetes Drosselventil umfaßt, wobei das Drosselventil genau einander gegenüber angeordnete Druckmittel umfaßt, zwischen denen der Schlauch anbringbar ist und die den Durchströmungsquerschnitt des Schlauches derart beeinflussen, daß sie in einer ersten Endlage den Durchströmungsquerschnitt des Schlauches unbeeinflußt lassen und in einer zweiten Endlage den Schlauch ganz zusammenpressen.

Ein Flußregulator dieser Art ist in dem Siemens-Manual "Servo Ventilator 300" gezeigt und ist hier dafür vorgesehen, den Gasfluß, der den Exspirationsschlauch eines Respirators/Ventilators während einer Ausatmungsphase durchströmt, zu regeln. Während einer Inspirationsphase wird der Schlauch mit Hilfe des einen Druckmittels, das am Ende einer Welle eines Solenoids angebracht ist, gegen das andere Druckmittel, das ein fester Anschlag ist, ganz zusammengepreßt. Da das bewegliche Druckmittel die eine Seite des Schlauches ständig angreift, entsteht an dieser Seite des Schlauches eine verhältnismäßig große Abnutzung. Außerdem ist der Durchmesser des Exspirationsschlauches und damit auch der Durchströmungsquerschnitt an der Klemmstelle im Vergleich zum Rest des Schlauches verhältnismäßig klein, damit das Drosselventil eine solche Kraft gegen den Schlauch ausüben kann, daß dieser ganz verschlossen werden kann.

Der Erfindung liegt die Aufgabe zugrunde, einen Flußregulator der eingangs genannten Art mit einem Drosselventil zu schaffen, das den Schlauch schont und das außerdem einen Schlauch mit einem verhältnismäßig großen Durchströmungsquerschnitt ganz zusammenpressen kann.

Diese Aufgabe ist erfindungsgemäß dadurch gelöst, daß die Druckmittel derart ausgebildet sind, daß sie bei einem Zusammenpressen des Schlauches diesen von beiden Seiten aktiv zusammendrücken. Hierdurch ist eine gleichmäßigere Verteilung der Abnutzung des Schlauches gegeben, was eine verhältnismäßig hohe Lebensdauer mitsichbringt.

Dadurch, daß die Kraft zum Zusammenpressen des Schlauches auf zwei Druckmittel verteilt werden kann, kann der Exspirationsschlauch in dem Bereich, in dem das Drosselventil arbeitet, einen im Verhältnis zu bekannten Schläuchen größeren Durchströmungsquerschnitt aufweisen, ohne notwendigerweise ein Drosselventil, das eine größere Klemmkraft als die bisher bekannten Drosselventile haben, zu verwenden. Dadurch, daß der Schlauch einen verhältnismäßig großen Durchströmungsquerschnitt besitzen kann, wird nunmehr der Atmungswiderstand für den Patienten kleiner.

In einer vorteilhaften Ausführungsform des Drosselventils nach der Erfindung wird vorgeschlagen, daß die Druckmittel derart ausgebildet sind, den Schlauch so zusammendrücken zu können, daß ein annähernd symmetrisches Zusammendrücken des Schlauches erreicht wird. Auf diese Weise ist sichergestellt, daß eine gleichmäßige Verteilung der Abnutzung des Schlauches in diesem Bereich erreicht wird.

In einer vorteilhaften Weiterentwicklung der Erfindung wird vorgeschlagen, daß das Drosselventil ein Drehsolenoid mit einem Magnetgehäuse und eine Welle, die im Verhältnis zum Magnetgehäuse drehbar ist, umfaßt, wobei das Magnetgehäuse und die Welle mit jeweils einem Druckmittel in Form von nach außen gerichteten Zapfen versehen ist, die so angeordnet sind, daß sie sich gegenseitig in einer Ebene bewegen und zwischen denen der Schlauch anbringbar ist, wobei das Drehsolenoid um eine Zentrumachse mit einer Halterung drehbar verbunden ist, wobei die Zentrumachse mit der Welle des Drehsolenoids zusammenfällt. Hierdurch ist ein im Aufbau verhältnismäßig einfaches und gleichzeitig elegantes Drosselventil gegeben, bei dem ein symmetrisches Zusammenpressen des Schlauches erreicht wird.

Ein Drehsolenoid der genannten Art ist in dem Handbuch "Solenoids Design Manual" der Fa. SHINDENGEN ELECTRIC MFG. CO. LTD. gezeigt und beschrieben.

In Verbindung mit der oben genannten Weiterentwicklung des Drosselventils nach der Erfindung wird vorgeschlagen, daß die Zapfen parallel miteinander und mit der Welle des Drehsolenoids verlaufen und daß der eine Zapfen mit der Welle über einen Arm, der senkrecht zur Welle verläuft, verbunden ist. Durch die Längsrichtung der Zapfen verläuft der Teil des Exspirationsschlauches, der zusammengepreßt werden soll, senkrecht zur Welle des Solenoids.

Die Zapfen können in einer weiteren Ausführungsform des Drosselventils nach der Erfindung senkrecht gegen die Welle des Drehsolenoids verlaufen.

Um zu verhindern, daß der Schlauch beim Zusammenpressen wegen der Zapfen eventuell verschoben wird, wird nach der Erfindung vorgeschlagen, daß jeder Zapfen mit jeweils einer Hülse versehen ist, deren Länge etwa der Länge des Zapfens entspricht, wobei die Hülsen um die Zapfen drehbar sind.

In Verbindung damit, daß die Zapfen senkrecht zur Welle des Drehsolenoids verlaufen, wird nach der Erfindung vorgeschlagen, daß die Hülsen auf den Zapfen achsial verschiebbar sind. Hierdurch wird vermieden, daß der Schlauch bei einem Zusammenpressen in der Längsrichtung der Hülsen verschoben wird.

In einer weiteren vorteilhaften Ausbildung des Drosselventils nach der Erfindung wird vorgeschlagen, daß das Drosselventil zwei einander gegenüber angeordnete Arme umfaßt, deren eines Ende um jeweils eine im Raum angeordnete feste Achse schwenkbar ist, und daß die Arme mittels Federmitteln danach streben, auseinander zu schwenken, und daß der Schlauch zwischen den Armen anbringbar ist und daß das Drosselventil außerdem ein Teil umfaßt, das mittels Druckmitteln verschiebbar ist und die freien Enden der Arme umgreift und derart betätigen kann, daß die Arme in einer ersten Endlage des Teils einen gegenseitigen Abstand aufweisen, der mindestens dem Außendurchmesser des Schlauches entspricht und in einer zweiten Endlage einen gegenseitigen Abstand aufweisen, der einem ganz zusammengepreßten Schlauch entspricht. Hierdurch wird ein im Aufbau sehr stabiles Drosselventil, das den Schlauch symmetrisch zusammenpreßt, erhalten.

Im Hinblick auf eine vorteilhafte Weiterentwicklung des zuletzt beschriebenen Drosselventils nach der Erfindung wird vorgeschlagen, daß das Teil im Profil V-förmig ausgebildet ist und mittels Druckmitteln in dessen Längsrichtung verschiebbar ist. Mit Längsrichtung ist hier die Richtung, in die die freien Enden der Schenkel des V-förmigen Teils zeigen, gemeint. Dies macht es möglich, daß das V-förmige Teil die freien Enden der beiden Arme umgreifen und betätigen kann.

In einer weiteren Ausbildung des Drosselventils nach der Erfindung wird vorgeschlagen, daß die gegeneinander gerichteten Seiten der Arme eine derartige Form aufweisen, daß wenigstens die Seitenstrecken, die in der zweiten Endlage gegen den Schlauch anliegen, in dieser Lage parallel miteinander verlaufen. Hierdurch ist gewährleistet, daß eine gleichmässige Abnutzung des Schlauches gegeben ist.

In einer konstruktiv einfachen Ausführungsform des Druckmittels nach der Erfindung wird vorgeschlagen, daß es ein Solenoid umfaßt, dessen Welle das Teil derart beeinflussen kann, daß das Teil in seiner Längsrichtung verschoben wird.

Das Druckmittel kann nach der Erfindung auch eine Kurvenscheibe umfassen, deren eines Ende das V-förmige Teil aufweist und deren anderes Ende keilförmig ausgebildet ist, wobei das Druckmittel ferner ein Solenoid umfaßt, dessen Welle eine Rolle beeinflussen kann, gegen das keilförmige Teil derart zu drücken, daß die Kurvenscheibe mit dem V-förmigen Teil in seiner Längsrichtung verschoben wird.

In einer weiteren vorteilhaften Ausführungsform des Drosselventils nach der Erfindung wird vorgeschlagen, daß es ein Druckmittel in Form eines ersten Armes umfaßt, der um eine im Raum feste erste Welle, die senkrecht zur Längsrichtung des Armes verläuft, drehbar ist, wobei die Welle etwa an der Mitte des Armes angeordnet ist und wobei das Drosselventil ferner ein gegen das drehbare Druckmittel mittels einer zweiten Welle mit Hilfe einer Druckvorrichtung verschiebbar angeordnetem Druckmittel in Form eines zweiten Armes umfaßt, wobei die zweite Welle senkrecht zur und genau bzw. etwa genau vor der ersten Welle angeordnet ist, wobei die Arme im Bereich deren einen Endes, zwischen denen der Schlauche anbringbar ist, in einer ersten Endlage des zweiten Armes einen gegenseitigen Abstand aufweisen, der mindestens dem Außendurchmesser des Schlauches und in einer zweiten Endlage einen gegenseitigen Abstand aufweisen, der einem ganz zusammengepreßten Schlauch entspricht. Hierdurch ist ein weiteres Drosselventil gegeben, dessen Druckmittel den Schlauch so zusammendrücken kann, daß ein annähernd symmetrisches Zusammenpressen erfolgt.

In einer vorteilhaften Weiterentwicklung des zuletztgenannten Drosselventils nach der Erfindung wird vorgeschlagen, daß die Arme im Bereich deren anderer Enden mit einer Distanz versehen sind, die die Arme mit einem Abstand trennt, der einem ganz zusammengepreßten Schlauch entspricht. Hierdurch ist sichergestellt, daß die Arme bei einem zwischen diesem angebrachten Schlauch in der zweiten erwähnten Endlage parallel und mit einem Abstand zueinander verlaufen, der einen ganz zusammengepreßtem Schlauch entspricht.

In einer vorteilhaften Ausführungsform der Distanz nach der Erfindung wird vorgeschlagen, daß sie eine Rolle ist, die an dem einen oder an dem anderen Arm angebracht ist. Auf diese Weise entsteht bei einer Verschiebung des zweiten Armes in Richtung des ersten Armes im Bereich dieser Enden eine kaum spührbare Reibung zwischen der Distanz und dem Arm.

Die erwähnte zweite Welle kann nach der Erfindung vorzugsweise eine Solenoidwelle sein.

Die Erfindung ist nachfolgend anhand mehrerer in den Zeichnungen dargestellten Ausführungsbeispielen näher erläutert. Es zeigen:

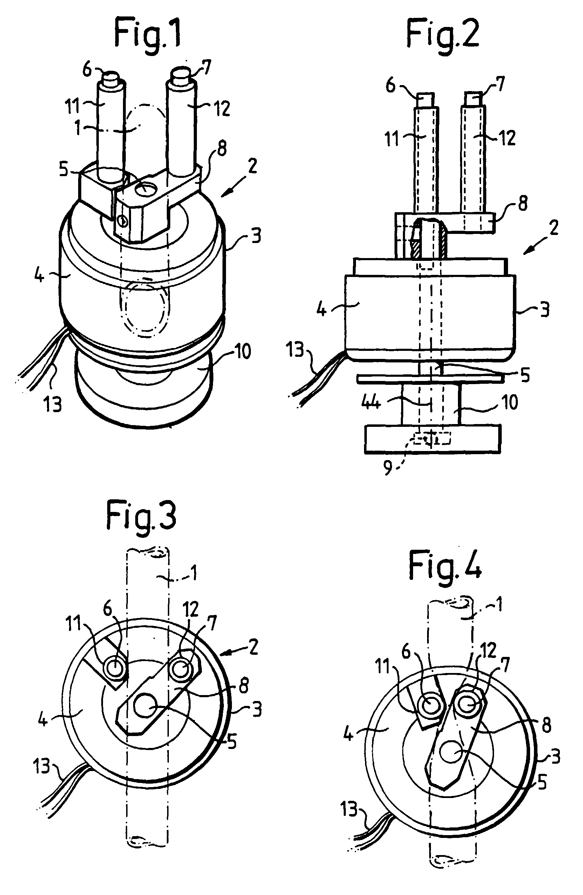

FIG. 1 - 11 Flußregulatoren in verschiedenen Ausführungen nach der Erfindung und in verschiedenen Lagen.

In der FIG. 1 ist ein Flußregulator nach der Erfindung, der vorzugsweise in Verbindung mit einem Respirator/Ventilator verwendet wird, in perspektivischer Ansicht gezeigt. Der Flußregulator umfaßt einen Exspirationsschlauch 1, der von einem Ausatmungsgas, dessen Fluß geregelt werden soll, durchströmt wird und bei dem das Ausatmungsgas von einem hier nicht gezeigten Patienten kommt. Der Schlauch 1 ist in der FIG. 1 mittels strichpunktierten Konturen gezeigt. Der Flußregulator umfaßt außerdem ein außerhalb des Schlauches 1 angeordnetes Drosselventil 2. Das Drosselventil 2 umfaßt ein Drehsolenoid 3 mit einem Magnetgehäuse 4 und eine Welle 5, die im Verhältnis zum Magnetgehäuse 4 drehbar ist. Das Magnetgehäuse 4 ist mit einem Zapfen 6, der mit Abstand zur Welle 5 angeordnet ist, versehene und die Welle 5 ist über einen Arm 8, der senkrecht zur Welle 5 verläuft, mit einem weiteren Zapfen 7, der mit dem Zapfen 6 parallel verläuft, und der durch den Arm 8 den gleichen Abstand zur Welle 5 wie der Zapfen 6 aufweist, verbunden. Die Zapfen 6 und 7, zwischen denen der Schlauch 1 anbringbar ist, können sich gegenseitig in einer Ebene bewegen. In diesem Ausführungsbeispiel verlaufen die Zapfen 6 und 7 parallel mit der Welle 5 des Drehsolenoids 3.

Das Drehsolenoid 3 ist über dessen Welle 5, die mit der Zentrumachse 44 des Drehsolenoids 3 zusammenfällt und über ein Lager 9 mit einer Halterung 10 drehbar verbunden. Dies ist in der FIG 2, die eine Seitenansicht des Flußregulators ist, gezeigt. In den FIG 1 und 2 ist gezeigt, daß jeder Zapfen 6 und 7 mit je einer Hülse 11, 12, deren Länge etwa der Länge der Zapfen 6, 7 entspricht, versehen ist. Die Hülsen 11, 12 sind außerdem um ihre jeweiligen Zapfen 6, 7 drehbar.

In der FIG 1 und in der FIG 3, die eine Draufsicht des Flußregulators ist, ist gezeigt, daß die Zapfen 6 und 7 in einer ersten Endlage einen derartigen Abstand zueinander haben, daß sie den Durchströmungsquerschnitt des Schlauches 1 unbeeinflußt lassen. Indem das Drehsolenoid 3 über Leitungen 13 mehr oder weniger Strom zugeführt wird, wird die Welle 5 im Verhältnis zum Magnetgehäuse 4 derart gedreht, daß der Arm 8 und gleichzeitig der Zapfen 7 in Richtung des Zapfens 6 des Magnetgehäuses 4 gedreht wird, wodurch der Zapfen 7 gegen die eine Seite des Schlauches 1 drückt. Da das Drehsolenoid 3 und die Welle 5, wie bereits beschreiben, in einer Halterung 10 drehbar angeordnet sind und da der in den Figuren gezeigte Exspirationsschlauch 1 am Respirator/Ventilator abnehmbar festgespannt ist, dreht sich das Magnetgehäuse 4 und dadurch auch der Zapfen 6 danach in Richtung des Zapfens 7, wobei der Zapfen 6 gegen die andere Seite des Schlauches 1 drückt.

Hierdurch wird ein annähernd symmetrisches Zusammenpressen des Schlauches erhalten, wobei der Durchströmungsquerschnitt nun so variiert werden kann, daß ein gewünschter Gasfluß erhalten wird.

In der FIG. 4, die eine Draufsicht des Flußregulators ist, ist gezeigt, daß das Drehsolenoid 3 so bemessen ist, eine derartige Kraft zu erzeugen, daß die Zapfen 6, 7 in einer zweiten Endlage den Schlauch 1 ganz zusammenpressen. Die bereits beschriebenen Hülsen 11, 12 der Zapfen 6, 7 verhindern nun, daß der Schlauch 1 beim Zusammenpressen verschoben wird. Die Zapfen 6, 7 halten nun während einer Einatmungsphase den Schlauch 1 in einer zusammengedrückten Lage. In einer nachfolgenden Ausatmungsphase wird die Stromzufuhr zum Drehsolenoid 3 ausgeschaltet, wobei die Welle 5 und dadurch auch der Arm 8 und der Zapfen 7 kraftlos werden und in ihre ursprünglichen Lagen zurückgehen, gleichzeitig damit, daß das Magnetgehäuse 4 und dadurch auch der Zapfen, wie es in den FIG 1 und 3 gezeigt ist, in seine ursprüngliche Lage zurückgeht.

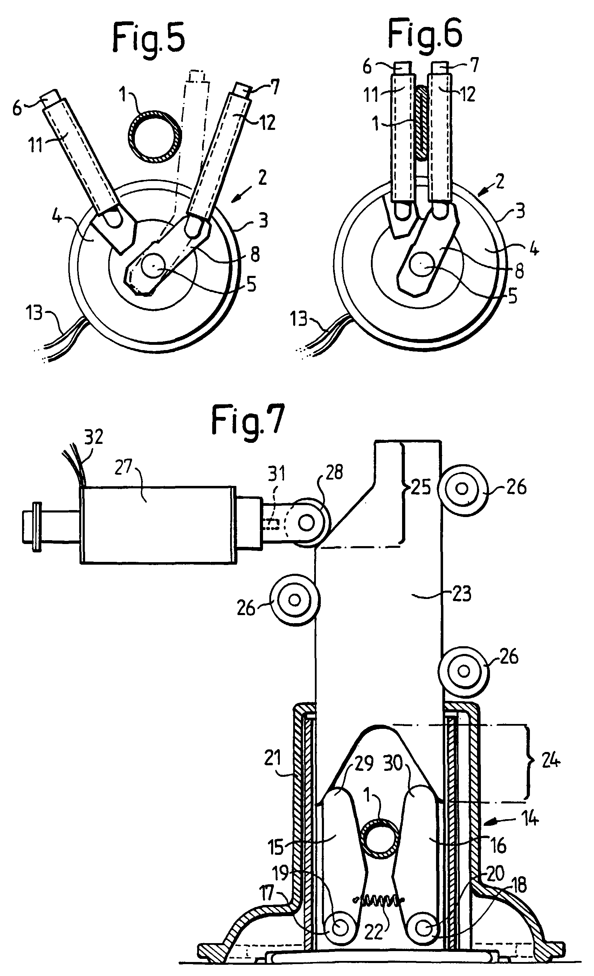

In der FIG 5 und 6 ist gezeigt, daß die Zapfen 6 und 7 des Flußregulators, statt mit der Welle 5 des Drehsolenoids 3 parallel zu verlaufen, so angeordnet sind, daß sie senkrecht zur Welle 5 verlaufen können. Im übrigen ist der Flußregulator in der gleichen Weise aufgebaut und arbeitet so wie es in Verbindung mit der FIG. 1 beschrieben worden ist. Bei einer solchen Ausführungsform verläuft der Exspirationsschlauch 1 parallel zur Welle 5 des Drehsolenoids 3. In der FIG 5 soll dargestellt werden, daß, wenn dem Drehsolenoid 3 Strom zugeführt wird, die Welle 5 im Verhältnis zum Magnetgehäuse 4 so gedreht wird, daß der Zapfen 7, wie es mit der strichpunktierten Version des Zapfens 7 gezeigt ist, gegen die Außenwand des Schlauches 1 anliegt. Da der Schlauch 1, wie bereits beschrieben, am Respirator/Ventilator abnehmbar befestigt ist, dient die Außenwand des Schlauches als ein temporärer Anschlag für den Zapfen 7. Hierdurch wird das Magnetgehäuse 4 mit dem Zapfen 6 dazu gezwungen, sich in Richtung der anderen Seite des Schlauches 1 zu bewegen, bis auch er gegen den Schlauch 1 anliegt. Danach erfolgt ein annähernd symmetrisches Zusammenpressen des Schlauches 1 bis er, wie in der FIG. 6 gezeigt, ganz zusammengepreßt ist. Damit in Verbindung mit einem Zusammenpressen vermieden wird, daß der Schlauch 1 in die Längsrichtung der Hülsen 11, 12 verschoben wird, sind die Hülsen 11, 12 auch am Zapfen 6, 7 achsial verschiebbar.

In der FIG. 7 ist ein weiterer Flußregulator nach der Erfindung in einer Seitenansicht gezeigt, bei dem das Drosselventil 14 eine im Längsschnitt gezeigte Halterung 21 und zwei einander gegenüber in der Halterung 21 angeordnete Arme 15, 16 umfaßt, zwischen denen der Schlauch 1 anbringbar ist. Die einen Enden 17, 18 der Arme 15, 16 sind um jeweils eine Welle 19, 20 schwenkbar gelagert. Die Wellen 19, 20 sind ihrerseits in der Halterung 21 befestigt. Die Arme 15, 16 streben auch danach, mittels Federmitteln, z.B. in Form einer Druckfeder 22, auseinanderzugehen. Das Drosselventil 14 umfaßt auch eine Kurvenscheibe 23, deren eines Ende aus einem V-förmigen Teil 24 besteht und deren anderes Ende 25 keilförmig ausgebildet ist. Die Kurvenscheibe 23 ist mittels Stützrollen 26 und mittels eines Solenoids 27, dessen Welle 31 mit einer Druckrolle 28 versehen ist, die, wie unten näher beschrieben wird, gegen die Peripherieoberfläche des keilförmigen Teils 25 anliegt, in ihrer Längsrichtung derart verschiebbar, daß das V-förmige Teil 25 in der Halterung 21 in der einen Endlage die freien Enden 29, 30 der Arme 15, 16 umgreifen kann. In dieser einen Endlage der Arme 15, 16 ist der Abstand zwischen diesen derart groß, daß es, wie es in der FIG. 7 gezeigt ist, den Durchströmungsquerschnitt des Schlauches 1 unbeeinflußt läßt.

Indem dem Solenoid 27 Strom über Leitungen 32 zugeführt wird, betätigt dessen Welle 31 die Druckrolle 28 derart, daß sie gegen die Peripherieoberfläche des keilförmigen Teils 25 der Kurvenscheibe 23 drückt, wobei das V-förmige Teil in seiner Längsrichtung verschoben wird. Dabei werden die freien Enden 29, 30 der Arme 15, 16 derart betätigt, daß die Arme 15, 16 aufeinander zukommen, wobei der Schlauch 1, der zwischen diesen angebracht ist, einem symmetrischen Zusammenpressen ausgesetzt wird.

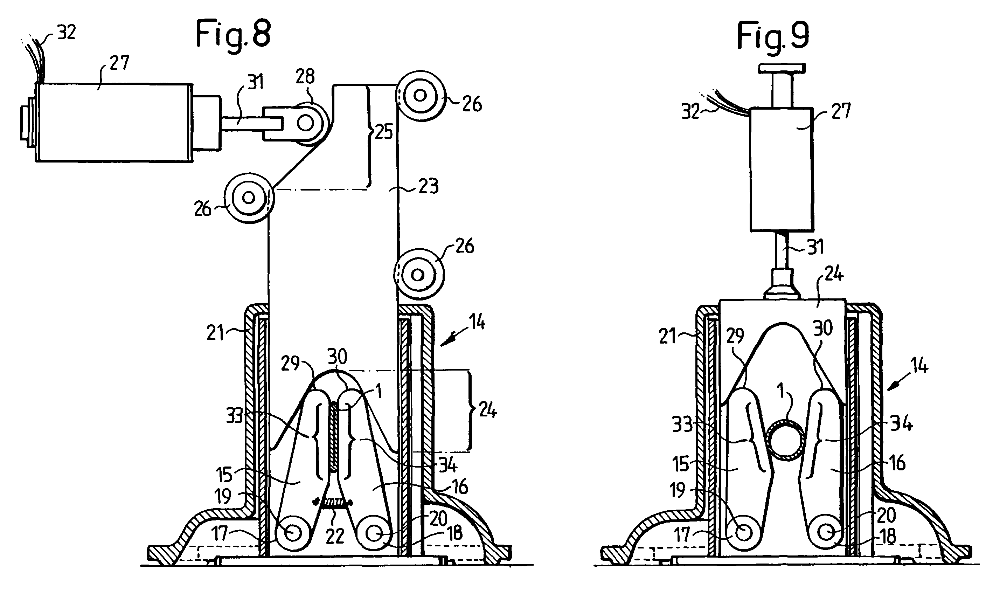

In der FIG. 8 ist eine Endlage gezeigt, in der die Arme 15, 16 den Schlauch 1 ganz zusammengepreßt haben. Wenn die Stromzufuhr zum Solenoid 27 ausgeschaltet wird, wird die Welle 31 mit ihrer Druckrolle 28 kraftlos, wodurch die Druckfeder 22 die Arme 15 und 16 auseinanderziehen kann. Hierbei wird die Kurvenscheiben 23 durch die Bewegung der Arme 15, 16 in ihre in der FIG 7 gezeigte ursprüngliche Lage zurückverschoben. In der FIG. 8 ist deutlich zu sehen, daß die gegeneinander gerichteten Seiten der Arme 15, 16 eine derartige Form aufweisen, daß die Seitenstrecken 33, 34, die in dieser Lage gegen den Schlauch 1 anliegen, parallel miteinander verlaufen.

In der FIG. 9 ist ein weiterer Flußregulator nach der Erfindung gezeigt, der eine große Ähnlichkeit mit dem Flußregulator, der in Verbindung mit den FIG. 7 und 8 beschrieben ist, hat. Der Unterschied ist lediglich, daß das Solenoid 27 über die Welle 31 direkt mit dem V-förmigen Teil 24 verbunden ist. Indem dem Solenoid 27 über Leitungen 32 Strom zugeführt wird, wird die Welle 31 beeinflußt, das V-förmige Teil 24 direkt so zu steuern, daß die Arme 15, 16 in beschriebener Weise eine gewünschte Lage erhalten und somit auf den Durchströmungsquerschnitt des Schlauches 1 einwirken.

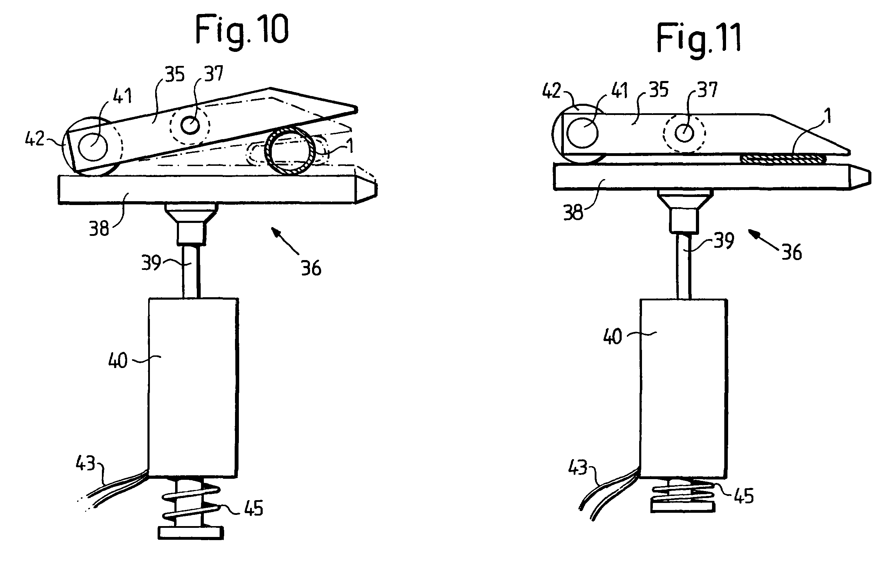

In den FIG. 10 und 11 ist ein weiterer Flußregulator nach der Erfindung gezeigt, bei dem das Drosselventil einen ersten Arm 35 umfaßt, der vorzugsweise um eine Welle 37, die in dem hier nicht dargestellten Respirator/Ventilator fest angeordnet ist, federnd drehbar ist. Die Welle 9, die senkrecht zur Längsrichtung des Armes 35 verläuft, ist etwa an der Mitte des Armes 35 angebracht. Das Drosselventil 36 umfaßt auch einen zweiten Arm 38, der mittels einer zweiten mit dem Arm 38 fest verbundenen Welle 39, die senkrecht zur und genau vor der ersten Welle 37 angeordnet ist, gegen den ersten Arm 35 verschoben werden kann. In diesem Ausführungsbeispiel ist die Welle 39 ein Teil des Solenoids 40. Der Arm 35 ist im Bereich dessen einen Endes mit einer um eine Welle 41 drehbaren Rolle 42 versehen, deren Peripherieoberfläche gegen den Arm 38 anliegt. Zwischen den Armen 35 und 38 im Bereich deren anderer Enden ist der Schlauch 1 anbringbar. Hier ist der Abstand zwischen den Armen 35, 38 so bemessen, daß er mindestens dem Außendurchmesser des Schlauches entspricht. Durch Zuführen von mehr oder weniger Strom über Leitungen 43 zum Solenoid 40 wird die Welle 39 des Solenoids 40 beeinflußt, den Arm 38 gegen die Rolle 42 derart zu drücken, daß der Arm 35 um seine Welle gedreht wird. Hierdurch wird der Abstand zwischen den Armen 35 und 37 im Bereich deren anderer Enden, in dem der Schlauch 1 angebracht ist, verringert, wobei der Schlauch 1 gleichzeitig von zwei gegenüberliegenden Seiten zusammengedrückt wird. Die strichpunktierten Konturen der Arme 35, 37 und des Schlauches 1 sollen ein solches Zusammenpressen des Schlauches 1 darstellen.

In der FIG. 11 ist gezeigt, daß der Arm 38 mit Hilfe der Welle 39 des Solenoids 40 die Rolle beeinflußt hat, den Arm 35 in eine Endlage, in der die Arme 35, 38 parallel miteinander verlaufen und in der der Schlauch 1 ganz zusammengepreßt ist, zu drehen. Die Parallelität der Arme 35, 38 wird erreicht, indem die Rolle 42 derart groß ist, daß sie die Arme 35, 38 mit einem Abstand voneinander trennt, der einem ganz zusammengepreßten Schlauch entspricht. Das Soleonid 40 hält nun, wie es in Verbindung mit dem bereits erwähnten Flußregulator beschrieben worden ist, während einer Einatmungsphase den Schlauch 1 in einer zusammengedrückten Lage. Bei einer nachfolgenden Ausatmungsphase wird die Stromzufuhr zum Solenoid 40 ausgeschaltet, wobei dessen Welle 39 kraftlos wird. Eine Druckfeder 45 sieht zu, daß die Welle 39 und damit auch der Arm 38 in die in der FIG. 10 gezeigte ursprüngliche Lage zurückverschoben wird. Auch die Arme 35, 38 dieses Flußregulators weisen, wie bereits beschrieben, ein solches Bewegungsmuster auf, daß ein annähernd symmetrisches Zusammenpressen des Schlauches 1 erreicht wird.

Das Wesentliche mit der Erfindung ist es, daß die hier beschriebenen Druckmittel in Form von Zapfen oder Armen so ausgebildet sind, daß sie bei einem Zusammenpressen des Exspirationsschlauches diesen von beiden Seiten aktiv derart zusammenpressen können, daß ein annähernd symmetrisches Zusammenpressen gegeben ist. Hierdurch wird ein Drosselventil erhalten, das für den Schlauch verhältnismäßig schonend ist. Da die Kraft, um den Schlauch ganz zusammenzupressen, auf zwei einander gegenüber angebrachte Druckmittel verteilt wird, kann ein Schlauch mit einem verhältnismäßig großen Durchströmungsquerschnitt verwendet werden, ohne ein Drosselventil, das eine größere Klemmkraft als die bisher bekannten Drosselventile aufweist, zu benutzen.

### Bezugszeichenliste

- 1: Exspirationsschlauch
- 2, 14, 36: Drosselventil
- 3: Drehsolenoid
- 4: Magnetgehäuse
- 5, 19, 20, 31, 37, 39, 41: Welle
- 6, 7: Zapfen
- 8: Arm
- 9: Lager
- 10, 21: Halterung
- 11, 12: Hülse
- 13, 32, 43: Leitung
- 15, 16, 35, 38: Arm, Druckmittel
- 17, 18: das eine Ende des Armes
- 22, 45: Druckfeder
- 23: Kurvenscheibe
- 24: V-förmiges Teil
- 25: keilförmiges Teil
- 26: Stützrolle
- 27, 40: Solenoid, Druckmittel
- 28: Druckrolle, Druckmittel
- 29, 30: das freie Ende des Armes
- 33, 34: Seitenstrecken
- 42: Rolle
- 44: Zentrumachse

## Patentansprüche

**1.** Flußregulator, vorzugsweise bei einem Respirator/Ventilator, bei dem der Flußregulator einen Schlauch, der von einem Medium, dessen Fluß geregelt werden soll, durchströmt wird, und ein außerhalb des Schlauches angeordnetes Drosselventil umfaßt, wobei das Drosselventil genau einander gegenüber angeordnete Druckmittel umfaßt, zwischen denen der Schlauch anbringbar ist und die den Durchströmungsquerschnitt des Schlauches derart beeinflussen, daß sie in einer ersten Endlage den Durchströmungsquerschnitt des Schlauches unbeeinflußt lassen und in einer zweiten Endlage den Schlauch ganz zusammenpressen, **dadurch gekennzeichnet,** daß die Druckmittel (6, 7, 15, 16, 35, 38) derart ausgebildet sind, daß sie bei einem Zusammenpressen des Schlauches (1), diesen von beiden Seiten aktiv zusammendrücken.

**2.** Flußregulator nach Anspruch 1, **dadurch gekennzeichnet,** daß die Druckmittel (6, 7, 15, 16, 35, 38) derart ausgebildet sind, den Schlauch (1) so zusammendrücken zu können, daß ein annähernd symmetrisches Zusammendrücken des Schlauches (1) erreicht wird.

**3.** Flußregulator nach Anspruch 1 oder 2, **dadurch gekennzeichnet,** daß das Drosselventil (2) ein Drehsolenoid (3) mit einem Magnetgehäuse (4) und eine Welle (5), die im Verhältnis zum Magnetgehäuse (4) drehbar ist, umfaßt, wobei das Magnetgehäuse (4) und die Welle (5) mit jeweils einem Druckmittel (6, 7) in Form von nach außen gerichteten Zapfen versehen ist, die so angeordnet sind, daß sie sich gegenseitig in einer Ebene bewegen und zwischen denen der Schlauch (1) anbringbar ist, wobei das Drehsolenoid (3) um eine Zentrumachse (44) mit einer Halterung (10) drehbar verbunden ist, wobei die Zentrumachse (44) mit der Welle (5) des Drehsolenoids (3) zusammenfällt.

**4.** Flußregulator nach Anspruch 3, **dadurch gekennzeichnet,** daß die Zapfen (6, 7) parallel miteinander und mit der Welle (5) des Drehsolenoids (3) verlaufen und daß der eine Zapfen (7) mit der Welle über einen Arm (8), der senkrecht zur Welle (5) verläuft, verbunden ist.

**5.** Flußregulator, nach einem der Ansprüche 3 bis 5, **dadurch gekennzeichnet,** daß jeder Zapfen (6, 7) mit jeweils einer Hülse (11, 12) versehen ist, deren Länge etwa der Länge des Zapfens (6, 7) entspricht, wobei die Hülsen (11, 12) um die Zapfen (6, 7) drehbar sind.

**7.** Flußregulator nach Anspruch 5 oder 6, **dadurch gekennzeichnet,** daß die Hülsen (11, 12) auf den Zapfen (6, 7) achsial verschiebbar sind.

**8.** Flußregulator nach Anspruch 1 oder 2, **dadurch gekennzeichnet,** daß das Drosselventil (14) zwei einander gegenüber angeordnete Arme (15, 16) umfaßt, deren eines Ende (17, 18) um jeweils eine im Raum angeordnete feste Achse (19, 20) schwenkbar ist, und daß die Arme (15, 16) mittels Federmitteln (22) danach streben, auseinander zu schwenken, und daß der Schlauch (1) zwischen den Armen (15, 16) anbringbar ist und daß das Drosselventil (14) außerdem ein Teil (23) umfaßt, das mittels Druckmitteln (27, 31) verschiebbar ist und die freien Enden (29, 30) der Arme (15, 16) umgreift und derart betätigen kann, daß die Arme (15, 16) in einer ersten Endlage des Teils (23) einen gegenseitigen Abstand aufweisen, der mindestens dem Außendurchmesser des Schlauches (1) entspricht und in einer zweiten Endlage einen gegenseitigen Abstand aufweisen, der einem ganz zusammengepreßten Schlauch (1) entspricht.

**9.** Flußregulator nach Anspruch 8, **dadurch gekennzeichnet,** daß das Teil (24) im Profil V-förmig ausgebildet ist und mittels Druckmitteln (27, 31) in dessen Längsrichtung verschiebbart ist.

**10.** Flußregulator nach Anspruch 8 oder 9, **dadurch gekennzeichnet,** daß die gegeneinander gerichteten Seiten der Arme (15, 16) eine derartige Form aufweisen, daß wenigstens die Seitenstrecken (33, 34), die in der zweiten Endlage gegen den Schlauch (1) anliegen, in dieser Lage parallel miteinander verlaufen.

**11.** Flußregulator nach einem der Ansprüche 8 bis 11, **dadurch gekennzeichnet,** daß das Druckmittel (27) ein Solenoid umfaßt, dessen Welle (31) das Teil (24) derart beinflussen kann, daß das Teil (24) in seiner Längsrichtung verschoben wird.

**12.** Flußregulator nach einem der Ansprüche 8 bis 11, **dadurch gekennzeichnet,** daß das Druckmittel eine Kurvenscheibe (23) umfaßt, deren eines Ende das V-förmige Teil (24) aufweist und deren anderes Ende keilförmig ausgebildet ist, wobei das Druckmittel ferner ein Solenoid (27) umfaßt, dessen Welle (31) eine Rolle (28) beinflussen kann, gegen das keilförmige Teil (25) derart zu drücken, daß die Kurvenscheibe (23) mit dem V-förmigen Teil (24) in seiner Längsrichtung verschoben wird.

**13.** Flußregulator nach Anspruch 1 oder 2, **dadurch gekennzeichnet,** das das Drosselventil ein Druckmittel in Form eines ersten Armes (35) umfaßt, der um eine im Raum feste erste Welle (37), die senkrecht zur Längsrichtung des Armes (35) verläuft, drehbar ist, wobei die Welle (37) etwa an der Mitte des Armes (35) angeordnet ist und wobei das Drosselventil ferner ein gegen das drehbare Druckmittel (35) mittels einer zweiten Welle (39) mit Hilfe einer Druckvorrichtung (40) verschiebbar angeordnetem Druckmittel in Form eines zweiten Armes (38) umfaßt, wobei die zweite Welle (39) senkrecht zur und genau bzw. etwa genau vor der ersten Welle (37) angeordnet ist, wobei die Arme (35, 38) im Bereich deren einen Endes, zwischen denen der Schlauch (1) anbringbar ist, in einer ersten Endlage des zweiten Armes (38) einen gegenseitigen Abstand aufweisen, der mindestens dem Außendurchmesser des Schlauches (1) und in einer zweiten Endlage einen gegenseitigen Abstand aufweisen, der einem ganz zusammengepreßten Schlauch (1) entspricht.

**14.** Flußregulator nach Anspruch 13, **dadurch gekennzeichnet,** daß die Arme (35, 38) im Bereich deren anderer Enden mit einer Distanz (42) versehen sind, die die Arme (35, 38) mit einem Abstand trennt, der einem ganz zusammengepreßten Schlauch (1) entspricht.

**15.** Flußregulator nach Anspruch 14, **dadurch gekennzeichnet,** daß die Distanz (42) eine Rolle ist, die an dem einen oder an dem anderen Arm (35, 38) angebracht ist.

**16.** Flußregulator nach einem der Ansprüche 13 bis 15, **dadurch gekennzeichnet,** daß die zweite Welle (39) eine Solenoidwelle ist.
